(19) Europäisches Patentamt
European Patent Office
Office européen des brevets

(11) **EP 4 092 438 A1**

(12) **EUROPEAN PATENT APPLICATION**

(43) Date of publication:
**23.11.2022 Bulletin 2022/47**

(21) Application number: **22174023.6**

(22) Date of filing: **18.05.2022**

(51) International Patent Classification (IPC):
**G01R 33/36** (2006.01)    **G01R 33/44** (2006.01)
**G01R 33/483** (2006.01)

(52) Cooperative Patent Classification (CPC):
**G01R 33/445; G01R 33/3628; G01R 33/4835;**
G01R 33/3607; G01R 33/3621; G01R 33/5611

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.05.2021 JP 2021083898**

(71) Applicant: **Canon Medical Systems Corporation Tochigi 324-0036 (JP)**

(72) Inventor: **TOMIHA, Sadanori Tochigi (JP)**

(74) Representative: **Marks & Clerk LLP 15 Fetter Lane London EC4A 1BW (GB)**

(54) **MAGNETIC RESONANCE IMAGING APPARATUS AND METHOD**

(57)    A magnetic resonance imaging apparatus (100) according to an embodiment includes a static magnetic field magnet (1), a plurality of radio frequency coils (120a, 120b), and a control unit (17a). The static magnetic field magnet (1) generates a static magnetic field having a magnetic field strength that changes spatially. The plurality of radio frequency coils (120a, 120b) receive a nuclear magnetic resonance signal generated from a subject by an influence of a radio frequency pulse transmitted to the subject, the subject being placed in the static magnetic field having a magnetic field strength that changes spatially. The control unit (17a) controls each of the plurality of radio frequency coils (120a, 120b) to receive the nuclear magnetic resonance signal at each of a plurality of frequencies tuned according to at least a distribution of the static magnetic field.

FIG.1

## Description

FIELD

Embodiments described herein relate generally to a magnetic resonance imaging apparatus and method.

BACKGROUND

In the related art, a magnetic resonance imaging (MRI) apparatus generates an image by transmitting a radio frequency (RF) pulse to a subject placed in a static magnetic field and receiving a nuclear magnetic resonance (NMR) signal generated from the subject by an influence of the RF pulse. The MRI apparatus includes an RF coil tuned to a predetermined resonance frequency in order to transmit an RF pulse and receive an NMR signal.

SUMMARY OF INVENTION

A magnetic resonance imaging apparatus according to an aspect of the present invention includes:

a static magnetic field magnet configured to generate a static magnetic field having a magnetic field strength that changes spatially;
a plurality of radio frequency coils configured to receive a nuclear magnetic resonance signal generated from a subject by an influence of a radio frequency pulse transmitted to the subject, the subject being placed in the static magnetic field having a magnetic field strength that changes spatially; and
a control unit configured to control each of the plurality of radio frequency coils to receive the nuclear magnetic resonance signal at each of a plurality of frequencies tuned according to at least a distribution of the static magnetic field.

The plurality of radio frequency coils may include a plurality of receiver coils respectively tuned to the plurality of frequencies.

The control unit may be configured to control each of the plurality of radio frequency coils to switch a frequency in receiving the nuclear magnetic resonance signal, on the basis of a frequency in transmitting the radio frequency pulse.

The control unit may be configured to control each of the plurality of radio frequency coils to switch the frequency in receiving the nuclear magnetic resonance signal, on the basis of a position of an imaging slice.

The control unit may be configured to control each of the plurality of radio frequency coils to sequentially transmit the radio frequency pulse at each of the frequencies and sequentially receive the nuclear magnetic resonance signal at each of the frequencies.

The control unit may be configured to control each of the plurality of radio frequency coils to transmit the radio frequency pulse in a band including the frequencies and simultaneously receive the nuclear magnetic resonance signal at each of the frequencies.

The plurality of radio frequency coils may include a plurality of transmitter coils configured to transmit the radio frequency pulse, and a plurality of receiver coils configured to receive the nuclear magnetic resonance signal, and

when one of the plurality of transmitter coils is configured to transmit the radio frequency pulse, the control unit may be configured to control a remaining one of the plurality of transmitter coils to be in a decoupled state.

The plurality of radio frequency coils may include a transmitter coil configured to be able to transmit the radio frequency pulse at the frequencies and a plurality of receiver coils configured to receive the nuclear magnetic resonance signal, and

when the transmitter coil is configured to transmit the radio frequency pulse, the control unit may be configured to control each of the plurality of receiver coils to be in a decoupled state.

When the plurality of receiver coils are configured to receive the nuclear magnetic resonance signal, the control unit may be configured to control the plurality of receiver coils to be able to simultaneously receive the nuclear magnetic resonance signal.

The static magnetic field having a magnetic field strength that changes spatially may be a static magnetic field that dominates a region where a magnetic field strength decays as a distance from the static magnetic field magnet increases.

The static magnetic field having a magnetic field strength that changes spatially may be a static magnetic field that dominates a region outside a uniform region where a magnetic field strength is uniform.

The static magnetic field having a magnetic field strength that changes spatially may be a static magnetic field that constantly forms a region where a magnetic field strength is not uniform.

The plurality of radio frequency coils each may receive the nuclear magnetic resonance signal generated from a same type of target atomic nucleus at a respective frequency of the plurality of frequencies.

The static magnetic field magnet may have a shape other than a shape encompassing the static magnetic field.

A magnetic resonance imaging method according to an aspect of the present invention includes:

generating a static magnetic field having a magnetic field strength that changes spatially, by using a static magnetic field magnet; and
controlling each of a plurality of radio frequency coils that receive a nuclear magnetic resonance signal generated from a subject by an influence of a radio frequency pulse transmitted to the subject, the subject being placed in the static magnetic field having a magnetic field strength that changes spatially, thereby receiving the nuclear magnetic resonance signal at each of a plurality of frequencies tuned according to at least a distribution of the static magnetic field.

BRIEF DESCRIPTION OF THE DRAWINGS

FIG. 1 is a diagram illustrating a configuration example of an MRI apparatus according to a first embodiment;

FIG. 2 is a diagram illustrating a static magnetic field generated by a static magnetic field magnet according to the first embodiment;

FIG. 3 is a diagram for explaining a reduction in the sensitivity of an RF coil associated with the first embodiment;

FIG. 4 is a diagram illustrating an example of an RF coil included in the MRI apparatus according to the first embodiment;

FIG. 5 is a diagram illustrating an example of an RF coil included in the MRI apparatus according to the first embodiment;

FIG. 6 is a diagram illustrating an example of an RF coil included in the MRI apparatus according to the first embodiment;

FIG. 7 is a diagram illustrating an example of the configuration of a transmission/reception system included in the MRI apparatus according to the first embodiment;

FIGs. 8A and 8B are a diagram illustrating an example of imaging performed by an imaging control function according to the first embodiment;

FIG. 9 is a diagram illustrating an example of an RF coil included in an MRI apparatus according to a second embodiment;

FIG. 10 is a diagram illustrating an example of the configuration of a transmission/reception system included in the MRI apparatus according to the second embodiment;

FIG. 11 is a diagram illustrating an example of the configuration of a transmission/reception system included in an MRI apparatus according to a third embodiment;

FIG. 12 is a diagram illustrating an example of the configuration of a transmission/reception system included in an MRI apparatus according to a fourth embodiment;

FIG. 13 is a diagram illustrating an example of the configuration of a transmission/reception system included in an MRI apparatus according to a fifth embodiment;

FIG. 14 is a diagram illustrating an example of the configuration of a transmission/reception system included in an MRI apparatus according to a sixth embodiment;

FIG. 15 is a diagram illustrating an example of the configuration of a transmission/reception system included in an MRI apparatus according to a seventh embodiment;

FIG. 16 is a diagram illustrating an example of the configuration of a transmission/reception system included in an MRI apparatus according to an eighth embodiment; and

FIG. 17 is a diagram illustrating an example of the configuration of a transmission/reception system included in an MRI apparatus according to a ninth embodiment.

DETAILED DESCRIPTION

A magnetic resonance imaging apparatus according to an embodiment includes a static magnetic field magnet, a plurality of radio frequency coils, and a control unit. The static magnetic field magnet generates a static magnetic field having a magnetic field strength that changes spatially. The plurality of radio frequency coils receive a nuclear magnetic resonance signal generated from a subject by an influence of a radio frequency pulse transmitted to the subject, the subject being placed in the static magnetic field having a magnetic field strength that changes spatially. The control unit controls each of the plurality of radio frequency coils to receive the nuclear magnetic resonance signal at each of a plurality of frequencies tuned according to at least a distribution of the static magnetic field.

Hereinafter, embodiments of an MRI apparatus and method according to the present application will be described in detail with reference to the drawings.

First Embodiment

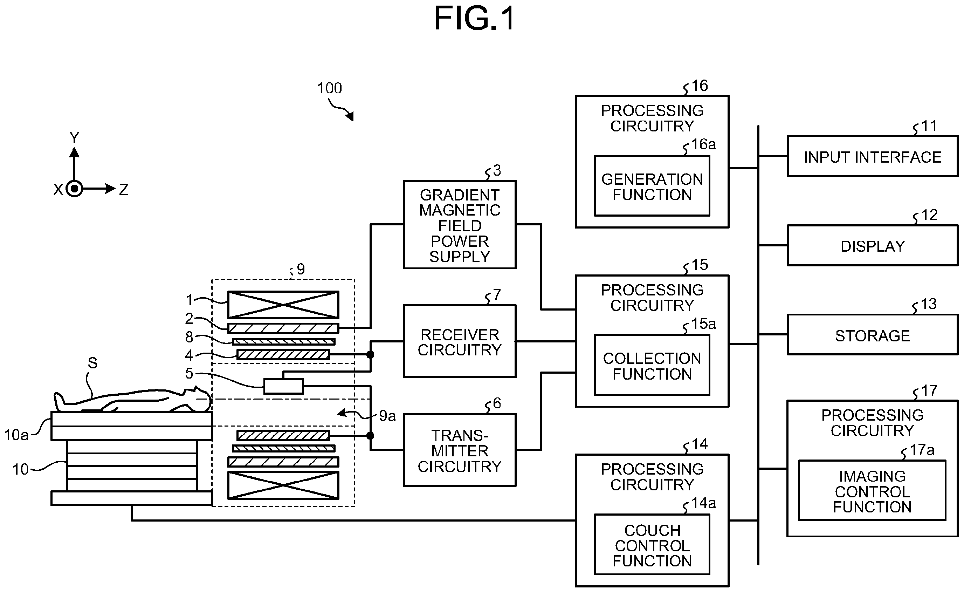

FIG. 1 is a diagram illustrating a configuration example of an MRI apparatus according to a first embodiment.

For example, as illustrated in FIG. 1, an MRI apparatus 100 includes a static magnetic field magnet 1, a gradient coil 2, a gradient magnetic field power supply 3, a whole body RF coil 4, a local RF coil 5, a transmitter circuitry 6, a receiver circuitry 7, an RF shield 8, a gantry 9, a couch 10, an input interface 11, a display 12, a storage 13, and processing circuitries 14 to 17.

The static magnetic field magnet 1 generates a static magnetic field in an imaging space where a subject S is arranged. Specifically, the static magnetic field magnet 1 is formed in a substantially hollow cylindrical shape (including a shape having an elliptical cross-section orthogonal to the central axis thereof), and generates a static magnetic field in the imaging space formed on the inner peripheral side thereof. For example, the static magnetic field magnet 1 is a superconducting magnet, a permanent magnet, or the like. The superconducting magnet described herein includes, for example, a vessel filled with a coolant such as liquid helium, and a superconducting coil immersed in the vessel.

The gradient coil 2 is arranged inside the static magnetic field magnet 1 and generates a gradient magnetic field in the imaging space where the subject S is arranged. Specifically, the gradient coil 2 is formed in a substantially hollow cylindrical shape (including a shape having an elliptical cross-section orthogonal to the central axis thereof), and has an X coil, a Y coil, and a Z coil respectively corresponding to an X axis, a Y axis, and a Z axis, which are orthogonal to one another. The X coil, the Y coil, and the Z coil generate a gradient magnetic field, which changes linearly along each axial direction, in the imaging space on the basis of current supplied from the gradient magnetic field power supply 3. The Z axis is set along a magnetic flux of the static magnetic field generated by the static magnetic field magnet 1. Furthermore, the X axis is set along a horizontal direction orthogonal to the Z axis, and the Y axis is set along a vertical direction orthogonal to the Z axis. The X axis, the Y axis, and the Z axis constitute a device coordinate system unique to the MRI apparatus 100.

The gradient magnetic field power supply 3 generates a gradient magnetic field in the imaging space by supplying current to the gradient coil 2. Specifically, the gradient magnetic field power supply 3 individually supplies current to the X coil, the Y coil, and the Z coil of the gradient coil 2, thereby generating, in the imaging space, a gradient magnetic field that changes linearly along each of a read out direction, a phase encode direction, and a slice direction orthogonal to one another. An axis along the read out direction, an axis along the phase encode direction, and an axis along the slice direction constitute a logical coordinate system for defining slice regions or volume regions to be imaged.

Specifically, gradient magnetic fields along the read out direction, the phase encode direction, and the slice direction are superimposed on the static magnetic field generated by the static magnetic field magnet 1, so that spatial position information is given to an NMR signal generated from the subject S. Specifically, the gradient magnetic field in the read out direction gives position information in the read out direction to the NMR signal by changing a frequency of the NMR signal according to a position in the read out direction. Furthermore, the gradient magnetic field in the phase encode direction gives position information in the phase encode direction to the NMR signal by changing a phase of the NMR signal according to a position in the phase encode direction. Furthermore, the gradient magnetic field in the slice direction gives position information in the slice direction to the NMR signal. For example, when an imaging region is the slice region (2D imaging), the gradient magnetic field in the slice direction is used for determining the direction and thickness of the slice region and the number thereof, and when an imaging region is the volume region (3D imaging), the gradient magnetic field in the slice direction is used for changing the phase of the NMR signal according to a position in the slice direction.

The whole body RF coil 4 is arranged on the inner peripheral side of the gradient coil 2, transmits an RF pulse to the subject S arranged in the imaging space, and receives an NMR signal generated from the subject S by an influence of the RF pulse. Specifically, the whole body RF coil 4 is formed in a substantially hollow cylindrical shape (including a shape having an elliptical cross-section orthogonal to the central axis thereof), and applies an RF magnetic field to the subject S arranged in the imaging space located on the inner peripheral side thereof, on the basis of an RF pulse supplied from the transmitter circuitry 6. Then, the whole body RF coil 4 receives the NMR signal generated from the subject S by an influence of the RF magnetic field, and outputs the received NMR signal to the receiver circuitry 7. For example, the whole body RF coil 4 is a birdcage type coil, or a transverse electromagnetic (TEM) coil. The whole body RF coil 4 may not have both a transmission function and a reception function, or may have only a transmission function.

The local RF coil 5 is arranged in the vicinity of the subject S at the time of imaging, transmits an RF pulse to the subject S arranged in the imaging space, and receives an NMR signal generated from the subject S by an influence of the RF pulse. Specifically, the local RF coil 5 is prepared for each portion of the subject S, arranged in the vicinity of a portion to be imaged when the subject S is imaged, and applies an RF magnetic field to the subject S on the basis of an RF pulse supplied from the transmitter circuitry 6. Then, the local RF coil 5 receives the NMR signal generated from the subject S by an influence of the RF magnetic field, and outputs the received NMR signal to the receiver circuitry 7. For example, the local RF coil 5 is a surface coil, or a phased array coil configured by combining a plurality of surface coils as coil elements. The local RF coil 5 may not have both a transmission function and a reception function, or may have only a reception function.

The transmitter circuitry 6 outputs an RF pulse, which corresponds to a resonance frequency (Larmor frequency) unique to a target atomic nucleus placed in the static magnetic field, to the whole body RF coil 4 or the local RF coil 5.

The receiver circuitry 7 generates NMR data on the basis of an NMR signal output from the whole body RF coil 4 or the local RF coil 5, and outputs the generated NMR data to the processing circuitry 15.

The RF shield 8 is arranged between the gradient coil 2 and the whole body RF coil 4, and shields the gradient coil 2 from an RF magnetic field generated by the whole body RF coil 4. Specifically, the RF shield 8 is formed in a substantially hollow cylindrical shape (including a shape having an elliptical cross-section orthogonal to the central axis of a cylinder), and is arranged in a space on the inner peripheral side of the gradient coil 2 to cover an outer peripheral surface of the whole body RF coil 4.

The gantry 9 has a hollow bore 9a formed in a substantially cylindrical shape (including a shape having an elliptical cross-section orthogonal to the central axis thereof), and accommodates the static magnetic field magnet 1, the gradient coil 2, the whole body RF coil 4, and the RF shield 8. Specifically, the gantry 9 accommodates the static magnetic field magnet 1, the gradient coil 2, the whole body RF coil 4, and the RF shield 8 in a state in which the whole body RF coil 4 is arranged on an outer peripheral side of the bore 9a, the RF shield 8 is arranged on an outer peripheral side of the whole body RF coil 4, the gradient coil 2 is arranged on an outer peripheral side of the RF shield 8, and the static magnetic field magnet 1 is arranged on an outer peripheral side of the gradient coil 2. The space in the bore 9a of the gantry 9 is the imaging space where the subject S is arranged at the time of imaging.

The couch 10 includes a couchtop 10a on which the subject S is placed, and moves the couchtop 10a on which the subject S is placed to the imaging space when the subject S is imaged. For example, the couch 10 is installed so that the longitudinal direction of the couchtop 10a is parallel to the central axis of the static magnetic field magnet 1.

The input interface 11 receives various instructions and input operations of various information from an operator. Specifically, the input interface 11 is connected to the processing circuitry 17, converts an input operation received from the operator into an electric signal, and outputs the electric signal to the processing circuitry 17. For example, the input interface 11 is implemented by a trackball for performing setting and the like of imaging conditions and region of interest (ROI), a switch button, a mouse, a keyboard, a touch pad that performs input operations in response to touch on the operation surface thereof, a touch screen in which a display screen and a touch pad are integrated, a non-contact input circuity using an optical sensor, a voice input circuity, and the like. In the present specification, the input interface 11 is not limited to one including physical operation parts such as a mouse and a keyboard. For example, an example of the input interface 11 includes an electric signal processing circuitry that receives an electric signal corresponding to an input operation from an external input device provided separately from the apparatus and outputs the electric signal to a control circuitry.

The display 12 displays various information. Specifically, the display 12 is connected to the processing circuitry 17, converts data of various information sent from the processing circuitry 17 into an electric signal for display, and outputs the electric signal. For example, the display 12 is implemented by a liquid crystal monitor, a cathode ray tube (CRT) monitor, a touch panel, or the like.

The storage 13 stores various data. Specifically, the storage 13 is connected to the processing circuitries 14 to 17, and stores various data input/output to/from each of the processing circuitries 14 to 17. For example, the storage 13 is implemented by a semiconductor memory element such as a random access memory (RAM) and a flash memory, a hard disk, an optical disk, or the like.

The processing circuitry 14 has a couch control function 14a. The couch control function 14a controls the operation of the couch 10 by outputting an electric signal for control to the couch 10. For example, the couch control function 14a receives an instruction for moving the couchtop 10a in the longitudinal direction, the vertical direction, and the leftright direction from the operator via the input interface 11, and operates a movement mechanism of the couchtop 10a of the couch 10 to move the couchtop 10a according to the received instruction.

The processing circuitry 15 has a collection function 15a. The collection function 15a collects k-space data by executing various pulse sequences. Specifically, the collection function 15a executes various pulse sequences by driving the gradient magnetic field power supply 3, the transmitter circuitry 6, and the receiver circuitry 7 according to sequence execution data output from the processing circuitry 17. The sequence execution data is data representing a pulse sequence, and is information that defines the timing at which the gradient magnetic field power supply 3 supplies current to the gradient coil 2 and the intensity of the supplied current, the timing at which the transmitter circuitry 6 supplies an RF pulse to the whole body RF coil 4 and the intensity of the supplied RF pulse, the timing at which the receiver circuitry 7 samples an NMR signal, and the like. Then, the collection function 15a receives NMR data from the receiver circuitry 7 as a result of executing the pulse sequence, and stores the NMR data in the storage 13. The NMR data stored in the storage 13 is given position information along each of the read out direction, the phase encode direction, and the slice direction by the aforementioned each gradient magnetic field, and thus is stored as k-space data representing a two-dimensional or three-dimensional k-space.

The processing circuitry 16 has a generation function 16a. The generation function 16a generates an image from the k-space data collected by the processing circuitry 15. Specifically, the generation function 16a reads the k-space data collected by the processing circuitry 15 from the storage 13, and performs reconstruction processing such as Fourier transform on the read k-space data, thereby generating a two-dimensional or three-dimensional image. Then, the generation function 16a stores the generated image in the storage 13.

The processing circuitry 17 has an imaging control function 17a. The imaging control function 17a performs various types of imaging by controlling each component of the MRI apparatus 100. Specifically, the imaging control function 17a displays, on the display 12, a graphical user interface (GUI) for receiving various instructions and input operations of various information from the operator, and controls each component of the MRI apparatus 100 according to the input operations received via the input interface 11.

For example, the imaging control function 17a generates sequence execution data on the basis of imaging conditions input by the operator, and outputs the generated sequence execution data to the processing circuitry 15, thereby allowing the processing circuitry 15 to collect k-space data. Furthermore, for example, the imaging control function 17a controls the processing circuitry 16 to reconstruct an image from the k-space data collected by the processing circuitry 15. Furthermore, for example, the imaging control function 17a reads an image from the storage 13 at the request of the operator, and allows the display 12 to display the read image.

The aforementioned processing circuitries 14 to 17 are implemented by a processor, for example. In such a case, processing functions of the processing circuitries are stored in the storage 13 in the form of computer programs that can be executed by a computer, for example. The processing circuitries read the computer programs from the storage 13 and execute the computer programs, respectively, thereby implementing processing functions corresponding to the executed computer programs. In other words, the processing circuitries having read the computer programs have the respective functions illustrated in the processing circuitries in FIG. 1.

In the above, an example in which each processing circuitry is implemented by a single processor has been described; however, embodiments are not limited thereto and each processing circuitry may be configured by combining a plurality of independent processors, and each of the processor may implement each processing function by executing a computer program. Furthermore, the processing functions of each processing circuitry may be implemented by being appropriately distributed or integrated into a single or a plurality of processing circuitries. Furthermore, in the example illustrated in FIG. 1, an example in which the single storage 13 stores a computer program corresponding to each processing function has been described; however, a plurality of storages may be arranged in a distributed manner and a processing circuitry may be configured to read a corresponding computer program from an individual storage.

So far, the configuration example of the MRI apparatus 100 according to the present embodiment has been described. Under such a configuration, in the present embodiment, the static magnetic field magnet 1 generates a static magnetic field, which has a magnetic field strength that changes spatially, in at least a part of the bore 9a that is an imaging space.

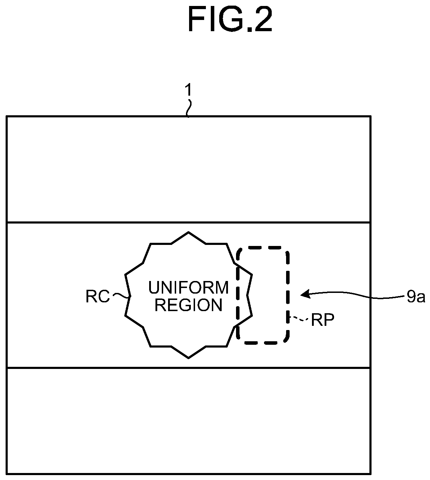

FIG. 2 is a diagram illustrating a static magnetic field generated by the static magnetic field magnet 1 according to the first embodiment.

For example, as illustrated in FIG. 2, for a static magnetic field generated by the static magnetic field magnet 1 formed in a cylindrical shape, the magnetic field strength is uniform in a region RC (hereinafter, uniform region) near the center in the bore 9a, but is not uniform in a region RP peripheral to the center and changes spatially.

In the present embodiment, the static magnetic field having a magnetic field strength that changes spatially can also be defined as, for example, a static magnetic field that dominates a region where the magnetic field strength decays as a distance from the static magnetic field magnet 1 increases, a region outside the uniform region where the magnetic field strength is uniform, a region where the magnetic field strength is not uniform, for example, a region where the magnetic field strength changes by 30 mT every 1 meter, and the like. That is, the static magnetic field having a magnetic field strength that changes spatially can also be referred to as a static magnetic field that constantly forms a region where the magnetic field strength is not uniform, regardless of whether to form the uniform region of the static magnetic field.

On the other hand, in general, an RF coil that receives an NMR signal is premised on the fact that the magnetic field strength of the static magnetic field is uniform and is tuned to a specific resonance frequency corresponding to the magnetic field strength in the uniform region. Therefore, in a region where the magnetic field strength of the static magnetic field changes spatially, the sensitivity of the RF coil may be reduced.

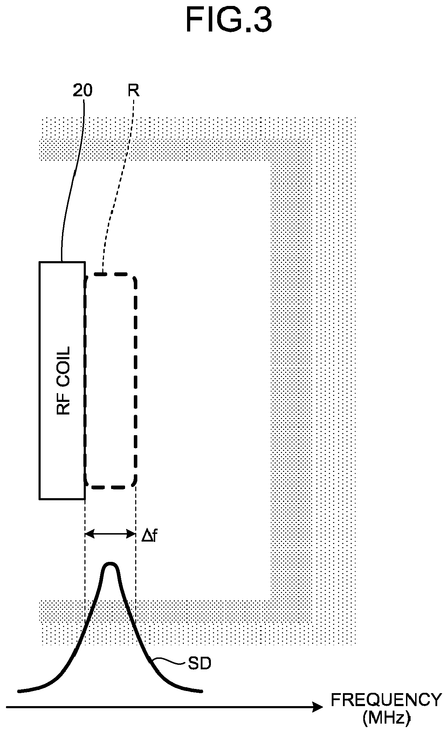

FIG. 3 is a diagram for explaining a reduction in the sensitivity of an RF coil associated with the first embodiment.

FIG. 3 conceptually illustrates a region where the magnetic field strength of a static magnetic field changes spatially, illustrates the distribution of the static magnetic field by a shaded pattern, and illustrates that the darker the shaded pattern, the stronger the magnetic field strength of the static magnetic field.

For example, as illustrated in FIG. 3, when the magnetic field strength is reduced as the distribution of the static magnetic field spreads, a resonance frequency is also reduced.

On the other hand, in general, an RF coil 20 has a sensitivity distribution in which the sensitivity is maximized at a specific resonance frequency and is reduced as a distance from the resonance frequency increases as in the curve SD illustrated in FIG. 3, and is adjusted to receive a signal in a band Af having a constant magnitude centered on the resonance frequency. Therefore, as illustrated in FIG. 3, a region R where the RF coil 20 can receive an NMR signal is limited to the range in which the resonance frequency falls within the band Δf, and the sensitivity of the RF coil 20 is reduced at a position where the resonance frequency shifts from the band Δf.

Therefore, the MRI apparatus 100 according to the present embodiment is configured to be able to improve the sensitivity of an RF coil when the magnetic field strength of a static magnetic field changes spatially.

Specifically, the MRI apparatus 100 includes an RF coil that transmits an RF pulse to a subject placed in a static magnetic field generated by the static magnetic field magnet 1 and having a magnetic field strength that changes spatially, and receives an NMR signal generated from the subject by an influence of the RF pulse. The RF coil may be the whole body RF coil 4 or the local RF coil 5. Alternatively, the RF coil may be a combination of the transmission function of the whole body RF coil 4 and the reception function of the local RF coil 5.

The imaging control function 17a of the processing circuitry 17 controls the RF coil to receive an NMR signal at each of a plurality of frequencies tuned according to the distribution of the static magnetic field generated by the static magnetic field magnet 1. The imaging control function 17a is an example of a control unit.

In the present embodiment, the MRI apparatus 100 includes a plurality of RF coils individually tuned to a plurality of frequencies, respectively. Then, the imaging control function 17a controls each of the RF coils to receive an NMR signal at each of the frequencies.

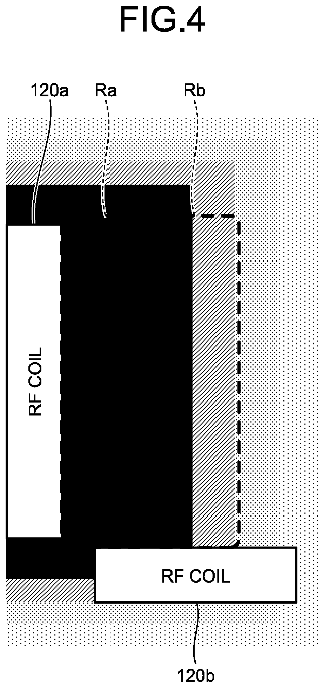

FIG. 4 to FIG. 6 are diagrams illustrating an example of an RF coil included in the MRI apparatus 100 according to the first embodiment.

FIG. 4 to FIG. 6 illustrate an example in which a magnetic field strength is reduced as the distribution of a static magnetic field spreads as in FIG. 3.

For example, as illustrated in FIG. 4, the MRI apparatus 100 includes a first RF coil 120a tuned to a frequency A and a second RF coil 120b tuned to a frequency B. The frequency A of the first RF coil 120a is tuned to a resonance frequency corresponding to the magnetic field strength of the static magnetic field in a first region Ra included in a range in which the static magnetic field is distributed. On the other hand, the frequency B of the second RF coil 120b is tuned to a resonance frequency corresponding to the magnetic field strength of the static magnetic field in a second region Rb included in a range in which the magnetic field strength of the static magnetic field is lower than that of the first region Ra.

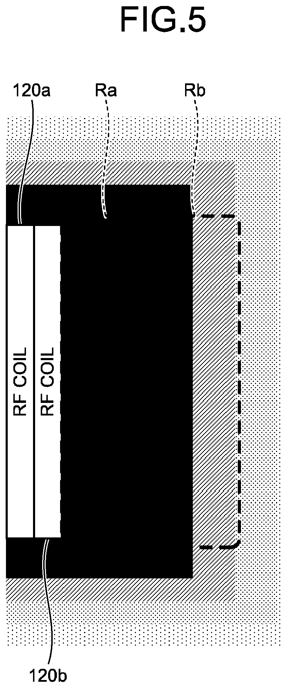

In such a case, the first RF coil 120a is arranged at a position where an NMR signal generated in the first region Ra can be received, and the second RF coil 120b is arranged at a position where an NMR signal generated in the second region Rb can be received. For example, as illustrated in FIG. 4, the first RF coil 120a and the second RF coil 120b are arranged so that a detection surface of the first RF coil 120a is orthogonal to a direction in which the distribution of the static magnetic field spreads and a detection surface of the second RF coil 120b is parallel to the direction in which the distribution of the static magnetic field spreads. Alternatively, for example, as illustrated in FIG. 5, the first RF coil 120a and the second RF coil 120b may be stacked and arranged so that their detection surfaces are orthogonal to the direction in which the distribution of the static magnetic field spreads.

Then, the imaging control function 17a controls the first RF coil 120a to receive an NMR signal at the frequency A and controls the second RF coil 120b to receive an NMR signal at the frequency B.

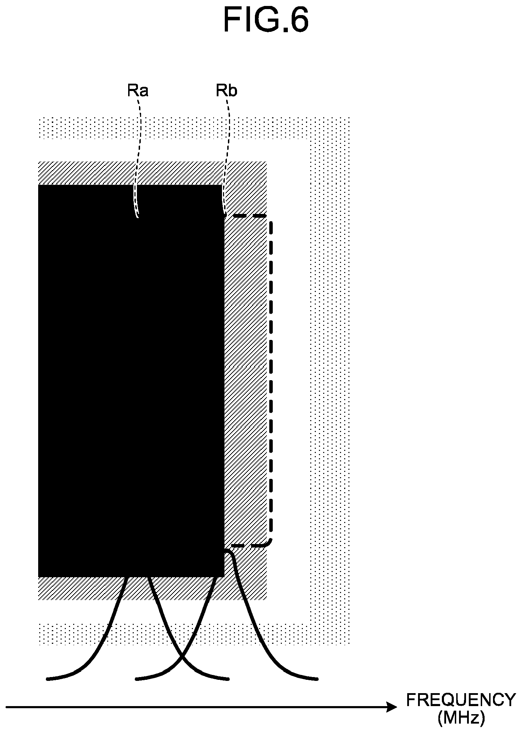

According to such a configuration, by using the two RF coils 120a and 120b tuned to the two frequencies A and B in a state in which they are independent of each other or are electromagnetically coupled to each other, for example, as illustrated in FIG. 6, NMR signals can be received from the two regions Ra and Rb along the direction in which the distribution of the static magnetic field spreads. This can widen a range in which NMR signals can be received along the direction in which the distribution of the static magnetic field spreads, and improve the sensitivity of the RF coil.

In the above, an example of receiving NMR signals at two frequencies by using two RF coils has been described; however, the number of RF coils and the number of frequencies are each not limited to two and may each be three or more. Thus, NMR signals can be received from three or more regions along the direction in which the distribution of the static magnetic field spreads, and a range in which the NMR signals can be received can be further widened.

For example, in the present embodiment, the MRI apparatus 100 includes, as the RF coils described above, a plurality of transmitter coils that transmit an RF pulse and a plurality of receiver coils that receive an NMR signal.

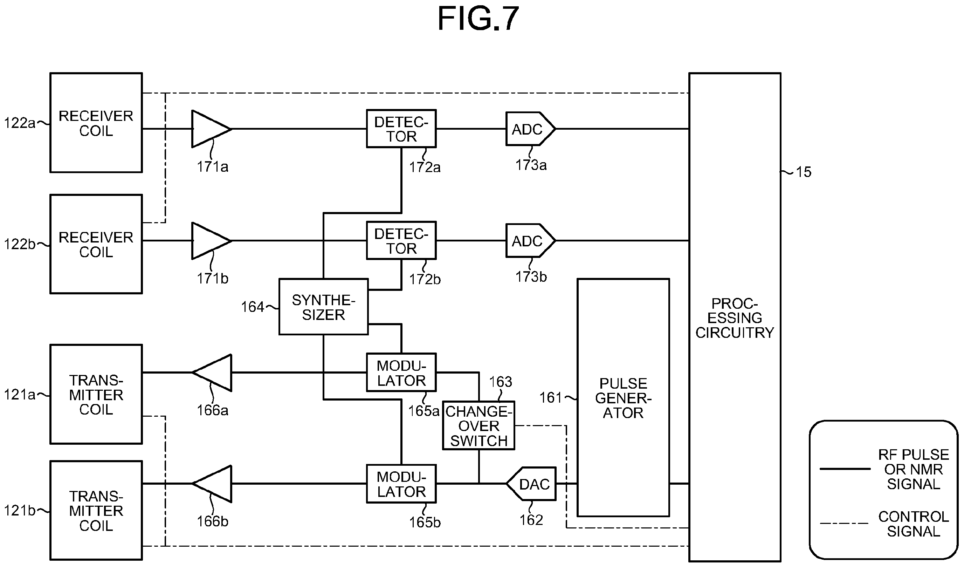

FIG. 7 is a diagram illustrating an example of the configuration of a transmission/reception system included in the MRI apparatus 100 according to the first embodiment.

For example, as illustrated in FIG. 7, the MRI apparatus 100 includes a first transmitter coil 121a and a first receiver coil 122a tuned to the frequency A, and a second transmitter coil 121b and a second receiver coil 122b tuned to the frequency B.

The first transmitter coil 121a transmits an RF signal having the frequency A to a subject according to a control signal transmitted from the imaging control function 17a of the processing circuitry 17 via the processing circuitry 15. The first receiver coil 122a receives an NMR signal, which is generated from the subject and has the frequency A, according to the control signal transmitted from the imaging control function 17a of the processing circuitry 17 via the processing circuitry 15. The second transmitter coil 121b transmits an RF signal having the frequency B to the subject according to the control signal transmitted from the imaging control function 17a of the processing circuitry 17 via the processing circuitry 15. The second receiver coil 122b receives an NMR signal, which is generated from the subject and has the frequency B, according to the control signal transmitted from the imaging control function 17a of the processing circuitry 17 via the processing circuitry 15.

Furthermore, the MRI apparatus 100 includes a pulse generator 161, a digital-to-analog converter (DAC) 162, a changeover switch 163, a synthesizer 164, a first modulator 165a, a second modulator 165b, a first RF amplifier 166a, and a second RF amplifier 166b. For example, these devices are included in the transmitter circuitry 6 illustrated in FIG. 1.

The pulse generator 161 generates an RF pulse waveform. The DAC 162 converts the RF pulse waveform generated by the pulse generator 161 from an analog signal to a digital signal, and outputs the digital signal. The changeover switch 163 outputs the digital signal, which is output from the DAC 162, to any one of the first modulator 165a and the second modulator 165b according to the control signal transmitted from the imaging control function 17a of the processing circuitry 17 via the processing circuitry 15. The synthesizer 164 generates and outputs an RF signal. The first modulator 165a converts the frequency of the RF signal output from the synthesizer 164 into the frequency A, and then modulates the RF signal with the waveform of the digital signal output from the changeover switch 163, thereby generating an RF pulse having the frequency A. The second modulator 165b converts the frequency of the RF signal output from the synthesizer 164 into the frequency B, and then modulates the RF signal with the waveform of the digital signal output from the changeover switch 163, thereby generating an RF pulse having the frequency B. The first RF amplifier 166a amplifies the RF pulse generated by the first modulator 165a and having the frequency A, and outputs the amplified pulse to the first transmitter coil 121a. The second RF amplifier 166b amplifies the RF pulse generated by the second modulator 165b and having the frequency B, and outputs the amplified pulse to the second transmitter coil 121b.

Furthermore, the MRI apparatus 100 includes a first preamplifier 171a, a second preamplifier 171b, a first detector 172a, a second detector 172b, a first analog-to-digital converter (ADC) 173a, and a second ADC 173b. For example, these devices are included in the receiver circuitry 7 illustrated in FIG. 1.

The first preamplifier 171a amplifies and outputs the NMR signal having the frequency A received by the first receiver coil 122a. The second preamplifier 171b amplifies and outputs the NMR signal having the frequency B received by the second receiver coil 122b. The first detector 172a converts the frequency of the RF signal output from the synthesizer 164 into the frequency A, detects the NMR signal output from the first preamplifier 171a by using the RF signal, and then outputs the detected NMR signal to the first ADC 173a. The second detector 172b converts the frequency of the RF signal output from the synthesizer 164 into the frequency B, detects the NMR signal output from the second preamplifier 171b by using the RF signal, and then outputs the detected NMR signal to the second ADC 173b. The first ADC 173a generates NMR data by converting the NMR signal output from the first detector 172a from an analog signal to a digital signal, and outputs the generated NMR data to the processing circuitry 15. The second ADC 173b generates NMR data by converting the NMR signal output from the second detector 172b from an analog signal to a digital signal, and outputs the generated NMR data to the processing circuitry 15.

Then, the imaging control function 17a controls the first transmitter coil 121a to transmit an RF pulse at the frequency A, and controls the first receiver coil 122a to receive an NMR signal at the frequency A. Furthermore, the imaging control function 17a controls the second transmitter coil 121b to transmit an RF pulse at the frequency B, and controls the second receiver coil 122b to receive an NMR signal at the frequency B.

At this time, when one of the first transmitter coil 121a and the second transmitter coil 121b transmits an RF pulse, the imaging control function 17a controls the other transmitter coil to be in a decoupled state. Furthermore, at this time, the imaging control function 17a controls both the first receiver coil 122a and the second receiver coil 122b to be in a decoupled state.

Furthermore, when the first receiver coil 122a and the second receiver coil 122b receive NMR signals, the imaging control function 17a controls the receiver coils to be able to receive the NMR signals at the same time. Furthermore, at this time, the imaging control function 17a controls both the first transmitter coil 121a and the second transmitter coil 121b to be in a decoupled state.

For example, the imaging control function 17a controls an element such as a PIN diode provided in each RF coil, thereby controlling each RF coil to perform transmission or reception at a desired frequency. Furthermore, for example, the imaging control function 17a controls the element such as a PIN diode provided in each RF coil and shifts a tuned frequency from the desired frequency, thereby controlling each RF coil to be in a decoupled state.

With such a configuration, the imaging control function 17a performs various types of imaging by controlling each of a plurality of RF coils to receive an NMR signal at each of a plurality of frequencies.

In such a case, on the basis of a frequency in transmitting an RF pulse, the imaging control function 17a controls the RF coil to switch a frequency in receiving an NMR signal.

Specifically, on the basis of a position of an imaging slice, the imaging control function 17a controls the RF coil to switch the frequency in receiving the NMR signal.

For example, the imaging control function 17a controls the RF coil to sequentially transmit an RF pulse at each of a plurality of frequencies and sequentially receive an NMR signal at each of the frequencies.

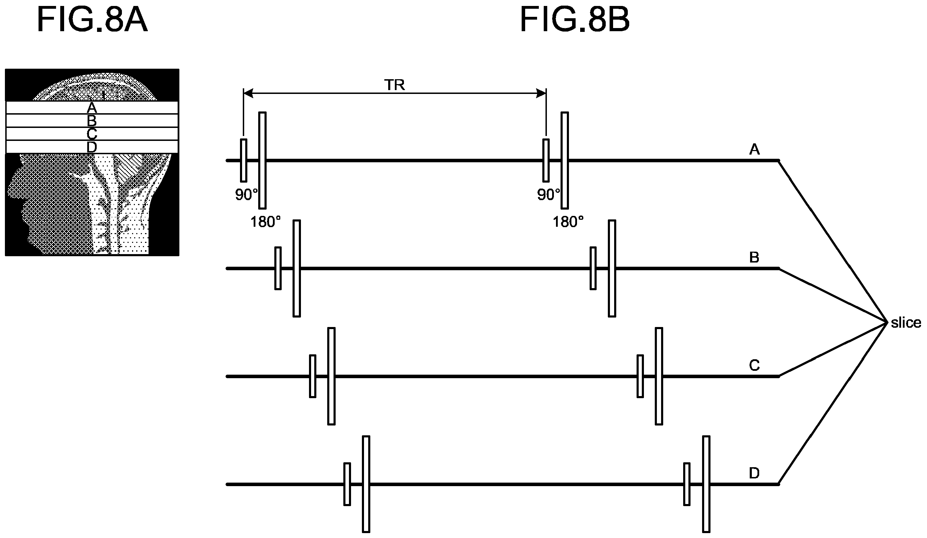

FIGs. 8A and 8B are a diagram illustrating an example of imaging performed by the imaging control function 17a according to the first embodiment.

For example, as illustrated in FIG. 8A, when a plurality of imaging slices A to D are imaged, it is assumed that the magnetic field strength of a static magnetic field generated by the static magnetic field magnet 1 changes along the slice direction. In such a case, the imaging control function 17a controls respective transmitter coils and respective receiver coils to transmit RF pulses and receive NMR signals at different frequencies for each of the imaging slices.

For example, as illustrated in FIG. 8B, the imaging control function 17a controls an RF coil (transmitter coil) to sequentially transmit an RF pulse (90° pulse) at an interval of repetition time (TR) for each of the imaging slices at a resonance frequency corresponding to the magnetic field strength of the static magnetic field at the position of each of the imaging slices. Then, the imaging control function 17a controls an RF coil (receiver coil) to sequentially receive an NMR signal at the same frequency as that of the RF pulse while changing the magnetic field strength of a gradient magnetic field in the phase encode direction for each TR.

At this time, for example, when a change in the magnetic field strength of the static magnetic field along the slice direction has a sufficient gradient, the gradient magnetic field in the slice direction may not be used. Alternatively, in order to correct the linearity of the change in the magnetic field strength of the static magnetic field, the gradient magnetic field in the slice direction may be supplementally used. In such a case, the imaging control function 17a controls an RF coil to perform transmission or reception at each of the frequencies tuned according to the distribution of the static magnetic field and the gradient magnetic field.

As described above, in the first embodiment, the static magnetic field magnet 1 generates a static magnetic field having a magnetic field strength that changes spatially. Furthermore, an RF coil transmits an RF pulse to a subject placed in the static magnetic field generated by the static magnetic field magnet 1 and having a magnetic field strength that changes spatially, and receives an NMR signal generated from the subject by an influence of the RF pulse. Then, the imaging control function 17a controls the RF coil to receive an NMR signal at each of a plurality of frequencies tuned according to at least the distribution of the static magnetic field.

Specifically, in the first embodiment, the MRI apparatus 100 includes a plurality of RF coils individually tuned to the frequencies, respectively. Then, the imaging control function 17a controls each of the RF coils to receive an NMR signal at each of the frequencies.

According to such a configuration, when the magnetic field strength of the static magnetic field changes spatially, a range in which NMR signals can be received can be widened by using the frequencies, and the sensitivity of the RF coil can be improved.

Although the first embodiment has been described above, embodiments of the MRI apparatus 100 according to the present application is not limited thereto. Hereinafter, other embodiments of the MRI apparatus 100 according to the present application will be described. In the following embodiments, points different from the first embodiment will be mainly described and description of contents common to the first embodiment will be omitted.

Second Embodiment

For example, in the aforementioned first embodiment, an example in which the MRI apparatus 100 includes a plurality of RF coils individually tuned to a plurality of frequencies, respectively, has been described; however, embodiments are not limited thereto. For example, the MRI apparatus 100 may include an RF coil configured to be tunable to each of a plurality of frequencies. Hereinafter, such an example will be described as a second embodiment.

In the present embodiment, the MRI apparatus 100 includes an RF coil configured to be tunable to a plurality of frequencies. Then, the imaging control function 17a switches a frequency of the RF coil to receive an NMR signal at each of the frequencies.

For example, the RF coil configured to be tunable to the frequencies is a double tuning coil or the like. For example, the imaging control function 17a switches the frequency of the RF coil by controlling an element such as a PIN diode provided in the RF coil and changing a pattern of a coil element included in the RF coil. Alternatively, for example, the imaging control function 17a switches the frequency of the RF coil by changing the capacity of a trimmer capacitor provided in the RF coil and shifting a tuned frequency.

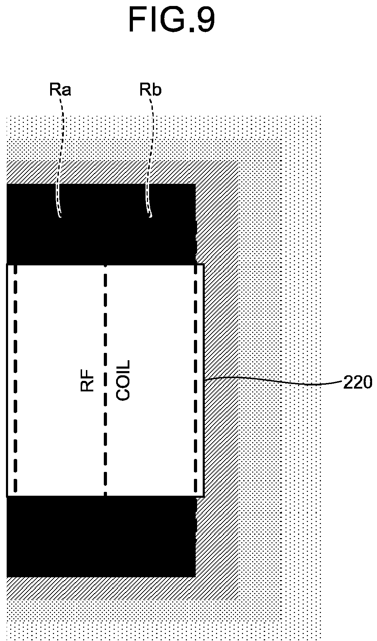

FIG. 9 is a diagram illustrating an example of an RF coil included in the MRI apparatus 100 according to the second embodiment.

FIG. 9 illustrates an example in which a magnetic field strength is reduced as the distribution of a static magnetic field spreads as in FIG. 3.

For example, as illustrated in FIG. 9, the MRI apparatus 100 includes an RF coil 220 configured to be tunable to two frequencies A and B. Then, the imaging control function 17a switches the frequency of the RF coil 220 to receive an NMR signal at each of the frequencies A and B.

Also in the present embodiment, the frequency A is set to a resonance frequency corresponding to the magnetic field strength of a static magnetic field in a first region Ra included in a range in which the static magnetic field is distributed. Furthermore, the frequency B is set to a resonance frequency corresponding to the magnetic field strength of the static magnetic field in a second region Rb included in a range in which the magnetic field strength of the static magnetic field is lower than that of the first region Ra.

According to such a configuration, by using the RF coil 220 tunable to the two frequencies A and B, NMR signals can be received from the two regions Ra and Rb along the direction in which the distribution of the static magnetic field spreads, as in the first embodiment. This can widen a range in which NMR signals can be received along the direction in which the distribution of the static magnetic field spreads, and improve the sensitivity of the RF coil.

In the above, an example of receiving an NMR signal at each frequency by using an RF coil tunable to two frequencies has been described; however, the number of frequencies is not limited to two and may be three or more. Thus, NMR signals can be received from three or more regions along the direction in which the distribution of the static magnetic field spreads, and a range in which the NMR signals can be received can be further widened.

For example, in the present embodiment, the MRI apparatus 100 includes, as the RF coils described above, one transmitter coil configured to be tunable to a plurality of frequencies and one receiver coil configured to be tunable to the frequencies.

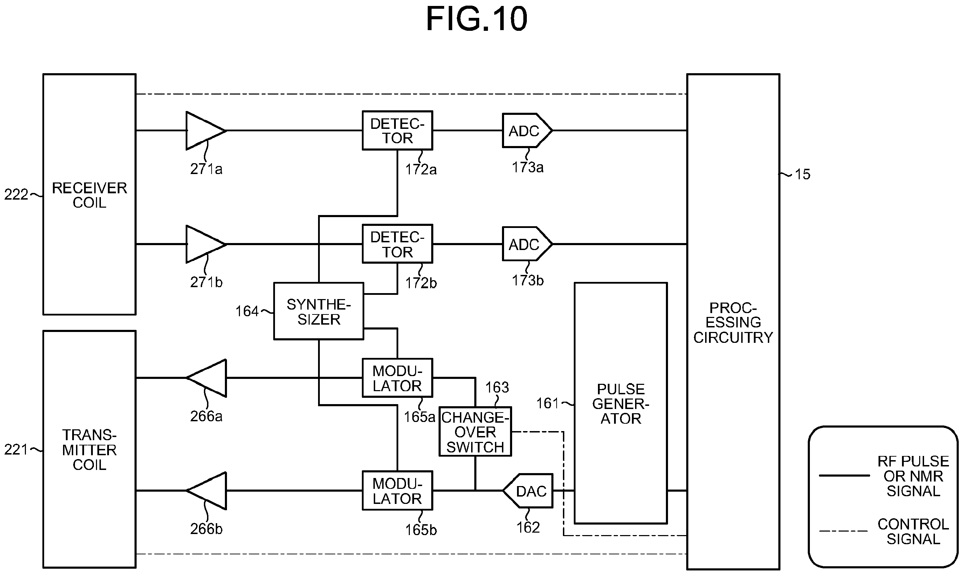

FIG. 10 is a diagram illustrating an example of the configuration of a transmission/reception system included in the MRI apparatus 100 according to the second embodiment.

For example, as illustrated in FIG. 10, the MRI apparatus 100 includes a transmitter coil 221 configured to be tunable to frequencies A and B and a receiver coil 222 configured to be tunable to the frequencies A and B.

The transmitter coil 221 transmits an RF signal having the frequency A or B to a subject according to a control signal transmitted from the imaging control function 17a of the processing circuitry 17 via the processing circuitry 15. The receiver coil 222 receives an NMR signal, which is generated from the subject and has the frequency A or B, according to the control signal transmitted from the imaging control function 17a of the processing circuitry 17 via the processing circuitry 15.

Furthermore, the MRI apparatus 100 includes a pulse generator 161, a DAC 162, a changeover switch 163, a synthesizer 164, a first modulator 165a, a second modulator 165b, a first RF amplifier 266a, and a second RF amplifier 266b. For example, these devices are included in the transmitter circuitry 6 illustrated in FIG. 1.

The pulse generator 161, the DAC 162, the changeover switch 163, the synthesizer 164, the first modulator 165a, and the second modulator 165b are the same as those in the first embodiment. The first RF amplifier 266a amplifies an RF pulse generated by the first modulator 165a and having the frequency A, and outputs the amplified pulse to the transmitter coil 221. The second RF amplifier 266b amplifies an RF pulse generated by the second modulator 165b and having the frequency B, and outputs the amplified pulse to the transmitter coil 221.

Furthermore, the MRI apparatus 100 includes a first preamplifier 271a, a second preamplifier 271b, a first detector 172a, a second detector 172b, a first ADC 173a, and a second ADC 173b. For example, these devices are included in the receiver circuitry 7 illustrated in FIG. 1.

The first preamplifier 271a amplifies and outputs an NMR signal having the frequency A received by the receiver coil 222. The second preamplifier 271b amplifies and outputs an NMR signal having the frequency B received by the receiver coil 222. The first detector 172a, the second detector 172b, the first ADC 173a, and the second ADC 173b are the same as those in the first embodiment.

Then, the imaging control function 17a controls the transmitter coil 221 to transmit an RF pulse at the frequency A, and controls the receiver coil 222 to receive an NMR signal at the frequency A. Furthermore, the imaging control function 17a controls the transmitter coil 221 to transmit an RF pulse at the frequency B, and controls the receiver coil 222 to receive an NMR signal at the frequency B.

With such a configuration, the imaging control function 17a performs various types of imaging by switching a frequency of a corresponding RF coil to receive an NMR signal at each of a plurality of frequencies.

In such a case, as in the first embodiment, on the basis of a frequency in transmitting an RF pulse, the imaging control function 17a controls the RF coil to switch the frequency in receiving the NMR signal.

Specifically, as in the first embodiment, on the basis of a position of an imaging slice, the imaging control function 17a controls the RF coil to switch the frequency in receiving the NMR signal.

For example, as in the first embodiment, the imaging control function 17a controls the RF coil to sequentially transmit an RF pulse at each of the frequencies and sequentially receive an NMR signal at each of the frequencies.

As described above, in the second embodiment, the MRI apparatus 100 includes an RF coil configured to be tunable to a plurality of frequencies, and the imaging control function 17a switches a frequency of the RF coil to receive an NMR signal at each of the frequencies.

According to such a configuration, as in the first embodiment, when the magnetic field strength of a static magnetic field changes spatially, a range in which NMR signals can be received can be widened by using the frequencies, and the sensitivity of the RF coil can be improved.

Third Embodiment

Furthermore, in the aforementioned first embodiment, an example in which the imaging control function 17a controls an RF coil to sequentially transmit an RF pulse at each of the frequencies and sequentially receive an NMR signal at each of the frequencies has been described; however, embodiments are not limited thereto. For example, the imaging control function 17a may transmit an RF pulse in a wide band including a plurality of frequencies. Hereinafter, such an example will be described as a third embodiment.

In the present embodiment, the imaging control function 17a controls an RF coil to transmit an RF pulse in a band including a plurality of frequencies and simultaneously receive NMR signals at the frequencies.

For example, in the present embodiment, the MRI apparatus 100 includes, as the RF coils described above, one transmitter coil that transmits an RF pulse and a plurality of receiver coils that receive NMR signals.

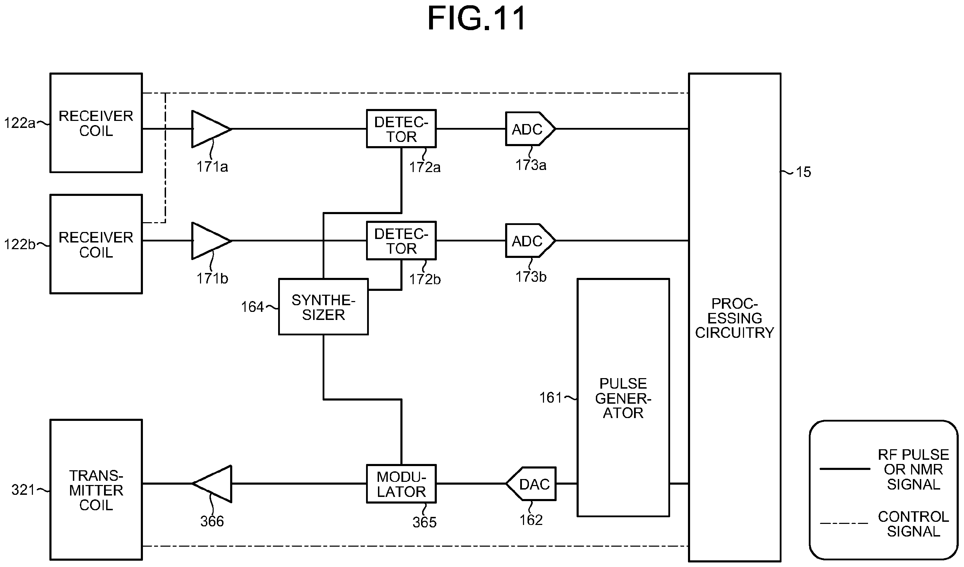

FIG. 11 is a diagram illustrating an example of the configuration of a transmission/reception system included in the MRI apparatus 100 according to the third embodiment.

For example, as illustrated in FIG. 11, the MRI apparatus 100 includes a transmitter coil 321 tuned to a band including frequencies A and B, a first receiver coil 122a tuned to the frequency A, and a second receiver coil 122b tuned to the frequency B.

The transmitter coil 321 transmits an RF signal to a subject in a band including the frequencies A and B according to a control signal transmitted from the imaging control function 17a of the processing circuitry 17 via the processing circuitry 15. The first receiver coil 122a and the second transmitter coil 121b are the same as those in the first embodiment.

Furthermore, the MRI apparatus 100 includes a pulse generator 161, a DAC 162, a modulator 365, and an RF amplifier 366. For example, these devices are included in the transmitter circuitry 6 illustrated in FIG. 1.

The pulse generator 161, the DAC 162, and the synthesizer 164 are the same as those in the first embodiment. The modulator 365 converts the frequency of an RF signal output from the synthesizer 164 into the frequency A, and then modulates the RF signal with a waveform of a digital signal output from the DAC 162, thereby generating an RF pulse in the band including the frequencies A and B. The RF amplifier 366 amplifies the RF pulse in the band including the frequencies A and B, which is generated by the modulator 365, and outputs the amplified pulse to the transmitter coil 321.

Furthermore, the MRI apparatus 100 includes a first preamplifier 171a, a second preamplifier 171b, a first detector 172a, a second detector 172b, a first ADC 173a, and a second ADC 173b. For example, these devices are included in the receiver circuitry 7 illustrated in FIG. 1.

The first preamplifier 171a, the second preamplifier 171b, the first detector 172a, the second detector 172b, the first ADC 173a, and the second ADC 173b are the same as those in the first embodiment.

Then, the imaging control function 17a controls the transmitter coil 321 to transmit the RF pulse in the band including the frequencies A and B. Furthermore, the imaging control function 17a controls the first receiver coil 122a to receive an NMR signal at the frequency A and controls the second receiver coil 122b to receive an NMR signal at the frequency B.

At this time, when the transmitter coil 321 transmits the RF pulse, the imaging control function 17a controls both the first receiver coil 122a and the second receiver coil 122b to be in a decoupled state.

Furthermore, when the first receiver coil 122a and the second receiver coil 122b receive NMR signals, the imaging control function 17a controls the receiver coils to be able to receive the NMR signals at the same time. Furthermore, at this time, the imaging control function 17a controls both the first transmitter coil 121a and the second transmitter coil 121b to be in a decoupled state.

With such a configuration, the imaging control function 17a performs various types of imaging by controlling the RF coil to transmit an RF pulse in a band including a plurality of frequencies and receive NMR signals at the frequencies.

For example, the imaging control function 17a performs parallel imaging by using a plurality of receiver coils tuned to different frequencies. For example, the receiver coils are a plurality of coil elements included in a phased-array coil.

In such a case, a frequency of each of the receiver coils is tuned to a resonance frequency corresponding to the magnetic field strength of a static magnetic field at the position of each coil. Then, the imaging control function 17a controls a transmitter coil to transmit an RF pulse in a band including the frequency of each of the receiver coils and controls the receiver coils to simultaneously receive NMR signals at the frequencies.

In the parallel imaging, an image is generated by synthesizing the NMR signals received by the receiver coils and is developed, resulting in the generation of an image with no wrapping. In general, a signal-to-noise ratio $(SNR)_{parallel}$ when using the parallel imaging is expressed by the following equation.

$$SNR_{parallel} = \frac{SNR}{g\sqrt{R}}$$

In the above equation, SNR is SNR when no parallel imaging is used, g denotes a g factor, and R denotes a double speed rate. Of these parameters, the g factor is an element that affects the image quality of an image. The higher the independence of the sensitivity distribution of each receiver coil, the smaller the value of the g factor, so that the image quality of a generated image is improved.

In this regard, in the present embodiment, by receiving NMR signals at a plurality of frequencies, a frequencydependent sensitivity distribution is generated in addition to a normal sensitivity distribution of receiver coils. Therefore, the independence of the sensitivity distribution of each receiver coil is further enhanced. As a consequence, the value of the g factor decreases, and the image quality of an image generated by the parallel imaging can be improved.

As described above, in the third embodiment, the imaging control function 17a controls an RF coil to transmit an RF pulse in a band including a plurality of frequencies and to simultaneously receive NMR signals at the frequencies.

According to such a configuration, as in the first embodiment, when the magnetic field strength of a static magnetic field changes spatially, a range in which NMR signals can be received can be widened by using a plurality of frequencies, and the sensitivity of the RF coil can be improved.

Furthermore, in the third embodiment, the image quality of an image generated by parallel imaging can be improved.

Fourth Embodiment

Furthermore, in the aforementioned second embodiment, an example in which a plurality of modulators and a plurality of detectors are used has been described; however, embodiments are not limited thereto. For example, in the configuration of the transmission/reception system illustrated in FIG. 10, when a modulator and a detector that can be switched to a plurality of frequencies are used, modulators and detectors may be shared, respectively. Hereinafter, such an example will be described as a fourth embodiment.

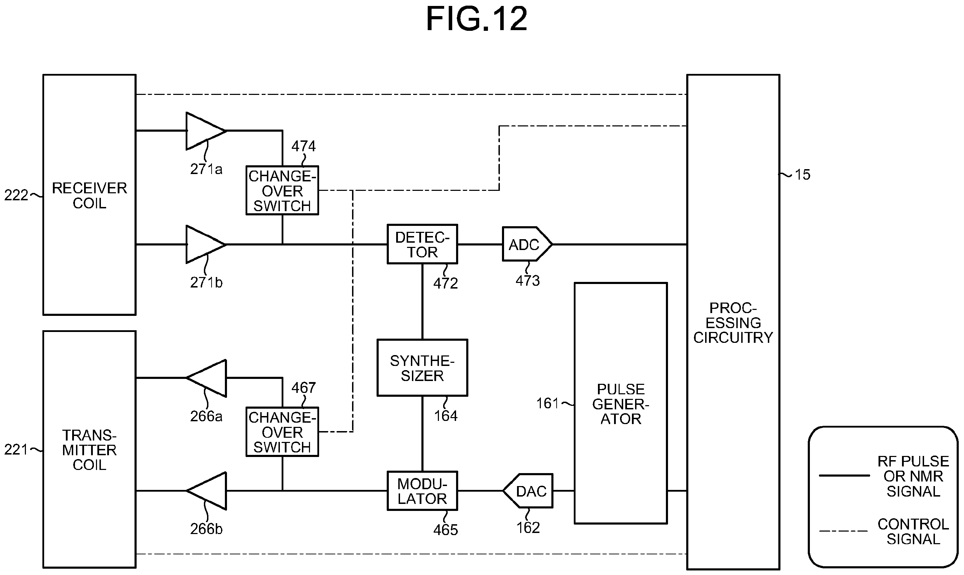

FIG. 12 is a diagram illustrating an example of the configuration of a transmission/reception system included in the MRI apparatus 100 according to the fourth embodiment.

For example, as illustrated in FIG. 12, the MRI apparatus 100 includes a transmitter coil 221 configured to be tunable to frequencies A and B and a receiver coil 222 configured to be tunable to the frequencies A and B.

The transmitter coil 221 and the receiver coil 222 are the same as those in the second embodiment.

Furthermore, the MRI apparatus 100 includes a pulse generator 161, a DAC 162, a synthesizer 164, a modulator 465, a changeover switch 467, a first RF amplifier 266a, and a second RF amplifier 266b. For example, these devices are included in the transmitter circuitry 6 illustrated in FIG. 1.

The pulse generator 161, the DAC 162, and the synthesizer 164 are the same as those in the first embodiment. The modulator 465 converts the frequency of an RF signal output from the synthesizer 164 into the frequencies A and B, and then modulates the RF signal with the waveform of a digital signal output from the DAC 162, thereby generating an RF pulse having the frequency A and an RF pulse having the frequency B. The changeover switch 467 outputs the RF pulse generated by the modulator 465 and having the frequency A to the first RF amplifier 266a or outputs the RF pulse generated by the modulator 465 and having the frequency B to the second RF amplifier 266b, according to a control signal transmitted from the imaging control function 17a of the processing circuitry 17 via the processing circuitry 15. The first RF amplifier 266a and the second RF amplifier 266b are the same as those in the second embodiment.

Furthermore, the MRI apparatus 100 includes a first preamplifier 271a, a second preamplifier 271b, a changeover switch 474, a detector 472, and an ADC 473. For example, these devices are included in the receiver circuitry 7 illustrated in FIG. 1.

The first preamplifier 271a and the second preamplifier 271b are the same as those in the second embodiment. The changeover switch 474 outputs, to the detector 472, an NMR signal output from the first preamplifier 271a and having the frequency A or an NMR signal output from the second preamplifier 271b and having the frequency B, according to the control signal transmitted from the imaging control function 17a of the processing circuitry 17 via the processing circuitry 15. The detector 472 converts the frequency of the RF signal output from the synthesizer 164 into the frequencies A and B, detects the NMR signal output from the changeover switch 474 by using the RF signal, and then outputs the detected NMR signal. The ADC 473 generates NMR data by converting the NMR signal output from the detector 472 from an analog signal to a digital signal, and outputs the generated NMR data to the processing circuitry 15.

Then, as in the second embodiment, the imaging control function 17a controls the transmitter coil 221 and the receiver coil 222.

Fifth Embodiment

Furthermore, in the aforementioned first embodiment, an example in which a plurality of modulators and a plurality of detectors are used as in the second embodiment has been described; however, embodiments are not limited thereto. For example, in the configuration of the transmission/reception system illustrated in FIG. 7, when a modulator and a detector that can be switched to a plurality of frequencies are used as in the fourth embodiment, modulators and detectors may be shared, respectively. Hereinafter, such an example will be described as a fifth embodiment.

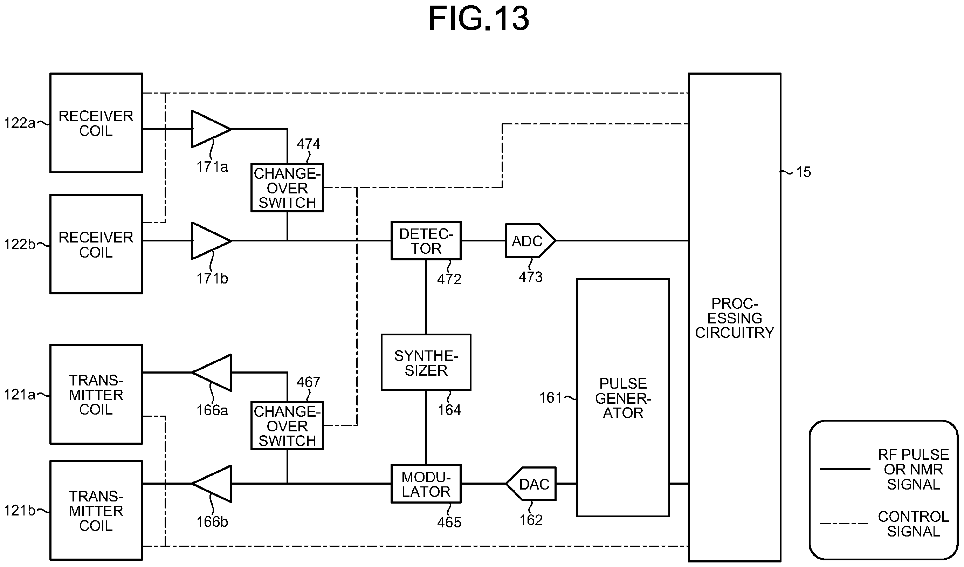

FIG. 13 is a diagram illustrating an example of the configuration of a transmission/reception system included in the MRI apparatus 100 according to the fifth embodiment.

For example, as illustrated in FIG. 13, the MRI apparatus 100 includes a first transmitter coil 121a and a first receiver coil 122a tuned to a frequency A and a second transmitter coil 121b and a second receiver coil 122b tuned to a frequency B.

The first transmitter coil 121a, the first receiver coil 122a, the second transmitter coil 121b, and the second receiver coil 122b are the same as those in the first embodiment.

Furthermore, the MRI apparatus 100 includes a pulse generator 161, a DAC 162, a synthesizer 164, a modulator 465, a changeover switch 467, a first RF amplifier 166a, and a second RF amplifier 166b. For example, these devices are included in the transmitter circuitry 6 illustrated in FIG. 1.

The pulse generator 161, the DAC 162, the synthesizer 164, the first RF amplifier 166a, and the second RF amplifier 166b are the same as those in the first embodiment. The modulator 465 and the changeover switch 467 are the same as those in the fourth embodiment.

Furthermore, the MRI apparatus 100 includes a first preamplifier 171a, a second preamplifier 171b, a changeover switch 474, a detector 472, and an ADC 473. For example, these devices are included in the receiver circuitry 7 illustrated in FIG. 1.

The first preamplifier 171a and the second preamplifier 171b are the same as those in the first embodiment. The changeover switch 474, the detector 472, and the ADC 473 are the same as those in the fourth embodiment.

Then, as in the first embodiment, the imaging control function 17a controls the first transmitter coil 121a, the second transmitter coil 121b, the first receiver coil 122a, and the second receiver coil 122b.

Sixth Embodiment

Furthermore, in the aforementioned first embodiment, an example in which a plurality of transmitter coils and a plurality of receiver coils are used has been described; however, embodiments are not limited thereto. For example, in the configuration of the transmission/reception system illustrated in FIG. 7, a plurality of transmitter/receiver coils may be used instead of a plurality of transmitter coils and a plurality of receiver coils. Hereinafter, such an example will be described as a sixth embodiment.

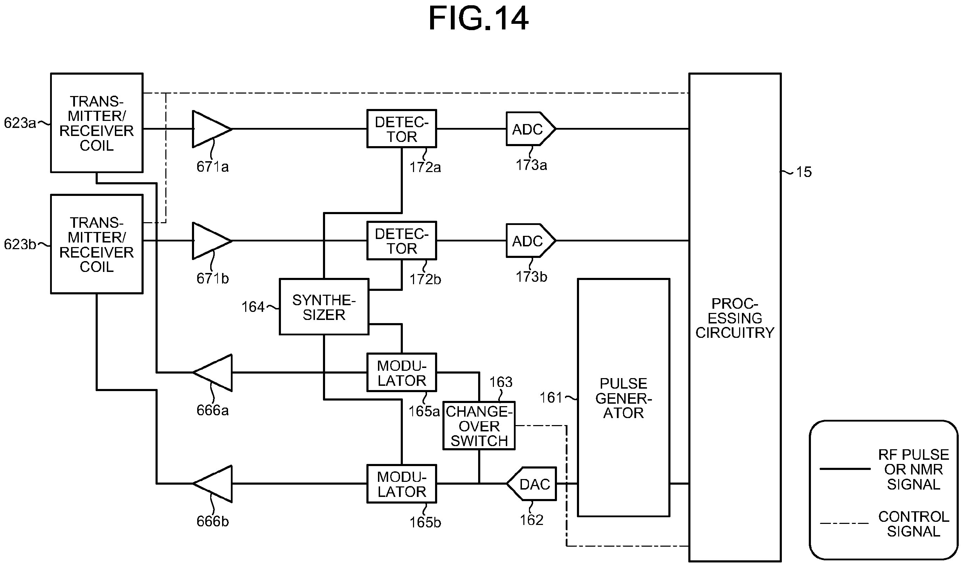

FIG. 14 is a diagram illustrating an example of the configuration of a transmission/reception system included in the MRI apparatus 100 according to the sixth embodiment.

For example, as illustrated in FIG. 14, the MRI apparatus 100 includes a first transmitter/receiver coil 623a tuned to a frequency A and a second transmitter/receiver coil 623b tuned to a frequency B.

The first transmitter/receiver coil 623a transmits an RF signal having the frequency A to a subject and receives an NMR signal generated from the subject and having the frequency A, according to a control signal transmitted from the imaging control function 17a of the processing circuitry 17 via the processing circuitry 15. The second transmitter/receiver coil 623b transmits an RF signal having the frequency B to the subject and receives an NMR signal generated from the subject and having the frequency B, according to the control signal transmitted from the imaging control function 17a of the processing circuitry 17 via the processing circuitry 15.

Furthermore, the MRI apparatus 100 includes a pulse generator 161, a DAC 162, a changeover switch 163, a synthesizer 164, a first modulator 165a, a second modulator 165b, a first RF amplifier 666a, and a second RF amplifier 666b. For example, these devices are included in the transmitter circuitry 6 illustrated in FIG. 1.

The pulse generator 161, the DAC 162, the changeover switch 163, the synthesizer 164, the first modulator 165a, and the second modulator 165b are the same as those in the first embodiment. The first RF amplifier 666a amplifies an RF pulse generated by the first modulator 165a and having the frequency A, and outputs the amplified pulse to the first transmitter/receiver coil 623a. The second RF amplifier 666b amplifies an RF pulse generated by the second modulator 165b and having the frequency B, and outputs the amplified pulse to the second transmitter/receiver coil 623b.

Furthermore, the MRI apparatus 100 includes a first preamplifier 671a, a second preamplifier 671b, a first detector 172a, a second detector 172b, a first ADC 173a, and a second ADC 173b. For example, these devices are included in the receiver circuitry 7 illustrated in FIG. 1.

The first preamplifier 671a amplifies and outputs an NMR signal having the frequency A received by the first transmitter/receiver coil 623a. The second preamplifier 671b amplifies and outputs an NMR signal having the frequency B received by the second transmitter/receiver coil 623b. The first detector 172a, the second detector 172b, the first ADC 173a, and the second ADC 173b are the same as those in the first embodiment.

Then, the imaging control function 17a controls the first transmitter/receiver coil 623a to transmit an RF pulse at the frequency A, and controls the first transmitter/receiver coil 623a to receive an NMR signal at the frequency A. Furthermore, the imaging control function 17a controls the second transmitter/receiver coil 623b to transmit an RF pulse at the frequency B, and controls the second transmitter/receiver coil 623b to receive an NMR signal at the frequency B.

At this time, when one of the first transmitter/receiver coil 623a and the second transmitter/receiver coil 623b transmits an RF pulse, the imaging control function 17a controls the other transmitter/receiver coil to be in a decoupled state.

Furthermore, when the first transmitter/receiver coil 623a and the second transmitter/receiver coil 623b receive NMR signals, the imaging control function 17a controls the transmitter/receiver coils to be able to receive the NMR signals at the same time.

Seventh Embodiment

Furthermore, in the aforementioned sixth embodiment, an example in which a plurality of modulators and a plurality of detectors are used as in the first embodiment has been described; however, embodiments are not limited thereto. For example, in the configuration of the transmission/reception system illustrated in FIG. 14, when a modulator and a detector that can be switched to a plurality of frequencies are used as in the fourth embodiment, modulators and detectors may be shared, respectively. Hereinafter, such an example will be described as a seventh embodiment.

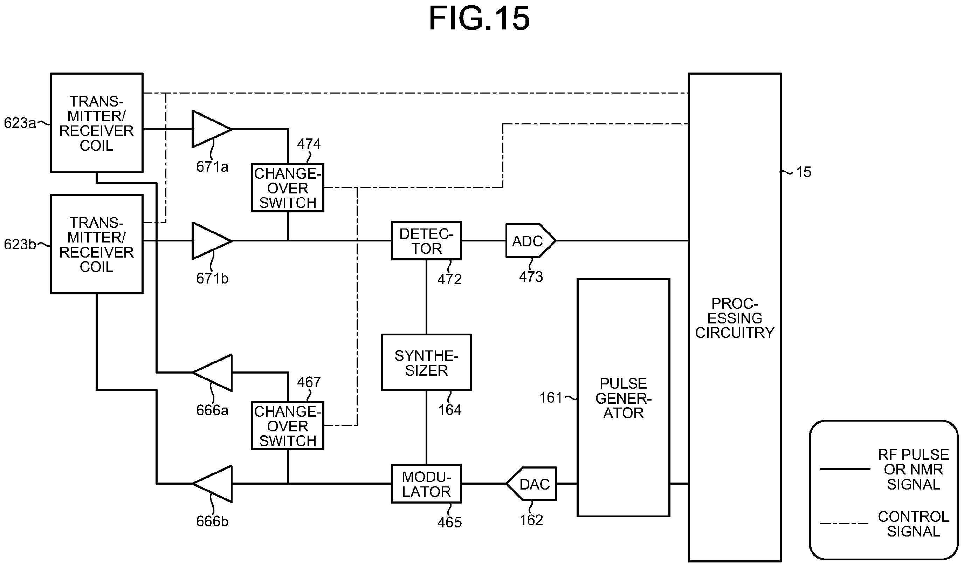

FIG. 15 is a diagram illustrating an example of the configuration of a transmission/reception system included in the MRI apparatus 100 according to the seventh embodiment.

For example, as illustrated in FIG. 15, the MRI apparatus 100 includes a first transmitter/receiver coil 623a tuned to a frequency A and a second transmitter/receiver coil 623b tuned to a frequency B.

The first transmitter/receiver coil 623a and the second transmitter/receiver coil 623b are the same as those in the

sixth embodiment.

Furthermore, the MRI apparatus 100 includes a pulse generator 161, a DAC 162, a synthesizer 164, a modulator 465, a changeover switch 467, a first RF amplifier 666a, and a second RF amplifier 666b. For example, these devices are included in the transmitter circuitry 6 illustrated in FIG. 1.

The pulse generator 161, the DAC 162, and the synthesizer 164 are the same as those in the first embodiment. The modulator 465 and the changeover switch 467 are the same as those in the fourth embodiment. The first RF amplifier 666a and the second RF amplifier 666b are the same as those in the sixth embodiment.

Furthermore, the MRI apparatus 100 includes a first preamplifier 671a, a second preamplifier 671b, a changeover switch 474, a detector 472, and an ADC 473. For example, these devices are included in the receiver circuitry 7 illustrated in FIG. 1.

The first preamplifier 671a and the second preamplifier 671b are the same as those in the sixth embodiment. The changeover switch 474, the detector 472, and the ADC 473 are the same as those in the fourth embodiment.

Then, as in the sixth embodiment, the imaging control function 17a controls the first transmitter/receiver coil 623a and the second transmitter/receiver coil 623b.

Eighth Embodiment

Furthermore, in the aforementioned second embodiment, an example in which one transmitter coil configured to be tunable to a plurality of frequencies and one receiver coil configured to be tunable to the frequencies are used has been described; however, embodiments are not limited thereto. For example, in the configuration of the transmission/reception system illustrated in FIG. 10, one transmitter/receiver coil configured to be tunable to a plurality of frequencies may be used instead of one transmitter coil and one receiver coil. Hereinafter, such an example will be described as an eighth embodiment.

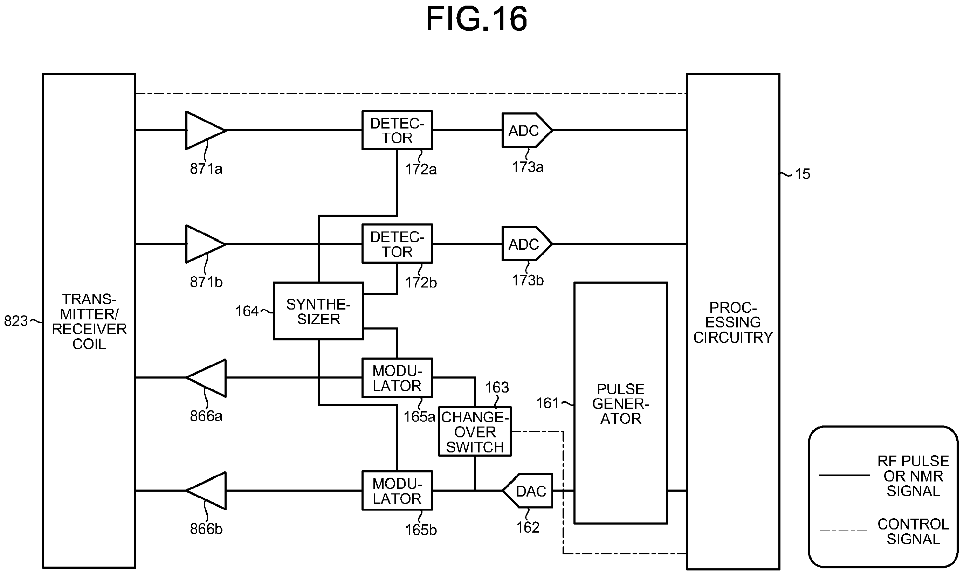

FIG. 16 is a diagram illustrating an example of the configuration of a transmission/reception system included in the MRI apparatus 100 according to the eighth embodiment.

For example, as illustrated in FIG. 16, the MRI apparatus 100 includes a transmitter/receiver coil 823 configured to be tunable to frequencies A and B.

The transmitter/receiver coil 823 transmits an RF signal having the frequency A or B to a subject according to a control signal transmitted from the imaging control function 17a of the processing circuitry 17 via the processing circuitry 15. Furthermore, the transmitter/receiver coil 823 receives an NMR signal, which is generated from the subject and has the frequency A or B, according to the control signal transmitted from the imaging control function 17a of the processing circuitry 17 via the processing circuitry 15.

Furthermore, the MRI apparatus 100 includes a pulse generator 161, a DAC 162, a changeover switch 163, a synthesizer 164, a first modulator 165a, a second modulator 165b, a first RF amplifier 866a, and a second RF amplifier 866b. For example, these devices are included in the transmitter circuitry 6 illustrated in FIG. 1.

The pulse generator 161, the DAC 162, the changeover switch 163, the synthesizer 164, the first modulator 165a, and the second modulator 165b are the same as those in the first embodiment. The first RF amplifier 866a amplifies an RF pulse generated by the first modulator 165a and having the frequency A, and outputs the amplified pulse to the transmitter/receiver coil 823. The second RF amplifier 866b amplifies an RF pulse generated by the second modulator 165b and having the frequency B, and outputs the amplified pulse to the transmitter/receiver coil 823.

Furthermore, the MRI apparatus 100 includes a first preamplifier 871a, a second preamplifier 871b, a first detector 172a, a second detector 172b, a first ADC 173a, and a second ADC 173b. For example, these devices are included in the receiver circuitry 7 illustrated in FIG. 1.

The first preamplifier 871a amplifies and outputs the NMR signal received by the transmitter/receiver coil 823 and having the frequency A. The second preamplifier 871b amplifies and outputs the NMR signal received by the transmitter/receiver coil 823 and having the frequency B.
The first detector 172a, the second detector 172b, the first ADC 173a, and the second ADC 173b are the same as those in the first embodiment.

Then, the imaging control function 17a controls the transmitter/receiver coil 823 to transmit an RF pulse at the frequency A, and controls the transmitter/receiver coil 823 to receive an NMR signal at the frequency A. Furthermore, the imaging control function 17a controls the transmitter/receiver coil 823 to transmit an RF pulse at the frequency B, and controls the transmitter/receiver coil 823 to receive an NMR signal at the frequency B.

Ninth Embodiment

Furthermore, in the aforementioned eighth embodiment, an example in which a plurality of modulators and a plurality of detectors are used as in the first embodiment has been described; however, embodiments are not limited thereto. For example, in the configuration of the transmission/reception system illustrated in FIG. 16, when a modulator and a detector that can be switched to a plurality of frequencies are used as in the fourth embodiment, modulators and detectors may be shared, respectively. Hereinafter, such an example will be described as a ninth embodiment.

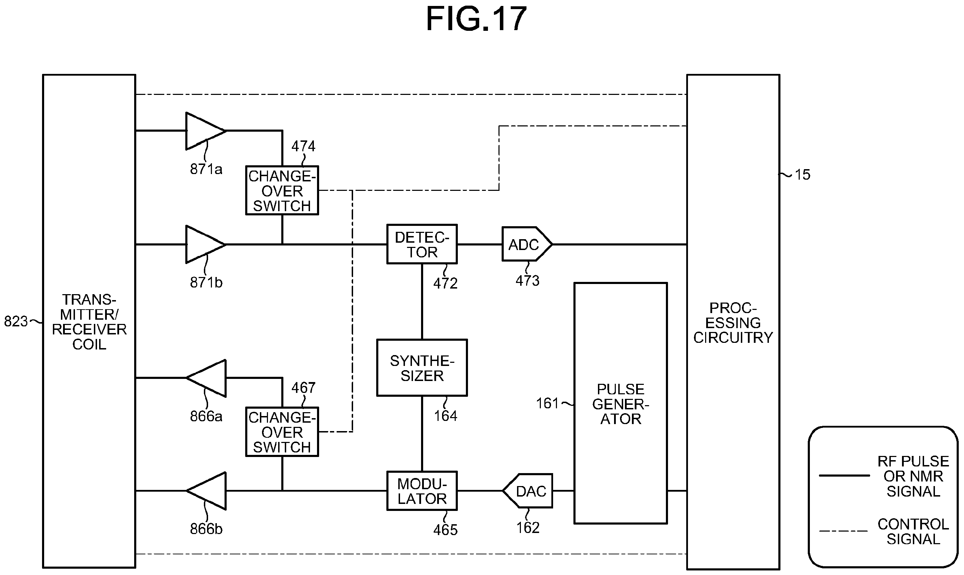

FIG. 17 is a diagram illustrating an example of the configuration of a transmission/reception system included in the MRI apparatus 100 according to the ninth embodiment.

For example, as illustrated in FIG. 17, the MRI apparatus 100 includes a transmitter/receiver coil 823 configured to be tunable to frequencies A and B.

The transmitter/receiver coil 823 is the same as that in the eighth embodiment.

Furthermore, the MRI apparatus 100 includes a pulse generator 161, a DAC 162, a synthesizer 164, a modulator 465, a changeover switch 467, a first RF amplifier 866a, and a second RF amplifier 866b. For example, these devices are included in the transmitter circuitry 6 illustrated in FIG. 1.

The pulse generator 161, the DAC 162, and the synthesizer 164 are the same as those in the first embodiment. The modulator 465 and the changeover switch 467 are the same as those in the fourth embodiment. The first RF amplifier 866a and the second RF amplifier 866b are the same as those in the eighth embodiment.

Furthermore, the MRI apparatus 100 includes a first preamplifier 871a, a second preamplifier 871b, a changeover switch 474, a detector 472, and an ADC 473. For example, these devices are included in the receiver circuitry 7 illustrated in FIG. 1.

The first preamplifier 871a and the second preamplifier 871b are the same as those in the eighth embodiment. The changeover switch 474, the detector 472, and the ADC 473 are the same as those in the fourth embodiment.

Then, as in the eighth embodiment, the imaging control function 17a controls the transmitter/receiver coil 823.

So far, the first to ninth embodiments have been described.

Among the aforementioned embodiments, in the first, third, and fifth to seventh embodiments, as a configuration for receiving an NMR signal at each of a plurality of frequencies, a plurality of RF coils (receiver coils or transmitter/receiver coils) individually tuned to each of the frequencies are used.

According to such a configuration, since each RF coil receives an NMR signal in a narrow band centered on a predetermined frequency, noise mixed in the NMR signal can be reduced as compared to the case of using RF coils (receiver coils or transmitter/receiver coils) configured to be tunable to the frequencies. This can improve the image quality of an image to be imaged as compared to the case of using the RF coils configured to be tunable to the frequencies. Furthermore, a reception system can be implemented with a simple circuit configuration as compared to the case of using the RF coils configured to be tunable to the frequencies.

Other Embodiments

In the aforementioned embodiments, the MRI apparatus 100, which has what is called a tunnel type structure in which each of the static magnetic field magnet 1, the gradient coil 2, and the whole body RF coil 4 is formed in a substantially cylindrical shape, has been described; however, embodiments are not limited thereto. For example, the technique disclosed in the present application can be applied in the same manner to an MRI apparatus having what is called an open structure in which a pair of static magnetic field magnets, a pair of gradient coils, and a pair of RF coils are arranged to face each other with an imaging space, where the subject S is arranged, interposed therebetween. That is, the technique disclosed in the present application can be applied to various MRI apparatuses as long as they are MRI apparatuses each including a static magnetic field magnet that generates a static magnetic field having a magnetic field strength that changes spatially in at least a part of an imaging space where a subject is arranged.

Furthermore, in the aforementioned embodiments, an example in which the control unit in the present specification is implemented by the imaging control function 17a of the processing circuitry 17 has been described; however, embodiments are not limited thereto. For example, the control unit in the present specification may implement the same function only by hardware, only by software, or a combination of hardware and software, in addition to the imaging control function 17a described in the embodiments.

Furthermore, in the above description, an example in which the "processor" reads a computer program corresponding to each processing function from the storage and executes the read computer program has been described; however, embodiments are not limited thereto. The term "processor", for example, means a circuit such as a central processing unit (CPU), a graphics processing unit (GPU), an application specific integrated circuit (ASIC), a programmable logic device (for example, a simple programmable logic device (SPLD)), a complex programmable logic device (CPLD), and a field programmable gate array (FPGA). When the processor is, for example, a CPU, the processor performs each processing function by reading and executing the computer program stored in the storage. On the other hand, when the processor is an ASIC, a corresponding processing function is directly incorporated in the circuit of the processor as a logic circuit instead of storing the computer program in the storage. Each processor of the present embodiment is not limited to being configured as a single circuit for each processor, and one processor may be configured by combining a plurality of independent circuits to perform processing functions thereof. Moreover, the components in FIG. 1 may be integrated into one processor to perform processing functions thereof.

The computer program executed by the processor is provided by being incorporated in advance in a read only memory (ROM), a storage, and the like. The computer program may be provided by being recorded on a computer readable storage medium, such as a CD (compact disc)-ROM, a flexible disk (FD), a CD-R (recordable), and a digital versatile disc (DVD), in a file format installable or executable in these devices. Furthermore, the computer program may be provided or distributed by being stored on a computer connected to a network such as the Internet and downloaded via the network. For example, the computer program is configured as a module including the aforementioned each functional unit. As actual hardware, the CPU reads and executes the computer program from the storage medium such as a ROM, so that each module is loaded on a main storage device and generated on the main storage device.

According to at least one embodiment described above, when the magnetic field strength of a static magnetic field changes spatially, the sensitivity of an RF coil can be improved.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims.

**Claims**

1. A magnetic resonance imaging apparatus (100) comprising:

   a static magnetic field magnet (1) configured to generate a static magnetic field having a magnetic field strength that changes spatially;
   a plurality of radio frequency coils (120a, 120b) configured to receive a nuclear magnetic resonance signal generated from a subject by an influence of a radio frequency pulse transmitted to the subject, the subject being placed in the static magnetic field having a magnetic field strength that changes spatially; and
   a control unit (17a) configured to control each of the plurality of radio frequency coils (120a, 120b) to receive the nuclear magnetic resonance signal at each of a plurality of frequencies tuned according to at least a distribution of the static magnetic field.

2. The magnetic resonance imaging apparatus (100) according to claim 1, wherein the plurality of radio frequency coils (120a, 120b) include a plurality of receiver coils (122a, 122b, 623a, 623b) respectively tuned to the plurality of frequencies.

3. The magnetic resonance imaging apparatus (100) according to claim 1 or 2, wherein the control unit (17a) is configured to control each of the plurality of radio frequency coils (120a, 120b) to switch a frequency in receiving the nuclear magnetic resonance signal, on the basis of a frequency in transmitting the radio frequency pulse.

4. The magnetic resonance imaging apparatus (100) according to any one of claims 1 to 3, wherein the control unit (17a) is configured to control each of the plurality of radio frequency coils (120a, 120b) to switch the frequency in receiving the nuclear magnetic resonance signal, on the basis of a position of an imaging slice.

5. The magnetic resonance imaging apparatus (100) according to any one of claims 1 to 4, wherein the control unit (17a) is configured to control each of the plurality of radio frequency coils (120a, 120b) to sequentially transmit the radio frequency pulse at each of the frequencies and sequentially receive the nuclear magnetic resonance signal at each of the frequencies.

6. The magnetic resonance imaging apparatus (100) according to any one of claims 1 to 4, wherein the control unit (17a) is configured to control each of the plurality of radio frequency coils (120a, 120b) to transmit the radio frequency pulse in a band including the frequencies and simultaneously receive the nuclear magnetic resonance signal at each of the frequencies.

7. The magnetic resonance imaging apparatus (100) according to any one of claims 1 to 6, wherein

   the plurality of radio frequency coils (120a, 120b) include a plurality of transmitter coils (121a, 121b) configured to transmit the radio frequency pulse, and a plurality of receiver coils (122a, 122b) configured to receive the nuclear magnetic resonance signal, and
   when one of the plurality of transmitter coils (121a, 121b) is configured to transmit the radio frequency pulse, the control unit (17a) being configured to control a remaining one of the plurality of transmitter coils (121a, 121b) to be in a decoupled state.

8. The magnetic resonance imaging apparatus (100) according to any one of claims 1 to 6, wherein

   the plurality of radio frequency coils (120a, 120b) include a transmitter coil (321) configured to be able to transmit the radio frequency pulse at the frequencies and a plurality of receiver coils (122a, 122b) configured to receive the nuclear magnetic resonance signal, and
when the transmitter coil (321) is configured to transmit the radio frequency pulse, the control unit (17a) being configured to control each of the plurality of receiver coils (122a, 122b) to be in a decoupled state.

9. The magnetic resonance imaging apparatus (100) according to claim 7 or 8, wherein, when the plurality of receiver coils (122a, 122b) are configured to receive the nuclear magnetic resonance signal, the control unit (17a) being configured to control the plurality of receiver coils to be able to simultaneously receive the nuclear magnetic resonance signal.

10. The magnetic resonance imaging apparatus (100) according to any one of claims 1 to 9, wherein the static magnetic field having a magnetic field strength that changes spatially is a static magnetic field that dominates a region where a magnetic field strength decays as a distance from the static magnetic field magnet (1) increases.

11. The magnetic resonance imaging apparatus (100) according to any one of claims 1 to 9, wherein the static magnetic field having a magnetic field strength that changes spatially is a static magnetic field that dominates a region outside a uniform region where a magnetic field strength is uniform.

12. The magnetic resonance imaging apparatus (100) according to any one of claims 1 to 9, wherein the static magnetic field having a magnetic field strength that changes spatially is a static magnetic field that constantly forms a region where a magnetic field strength is not uniform.

13. A magnetic resonance imaging method comprising:

generating a static magnetic field having a magnetic field strength that changes spatially, by using a static magnetic field magnet (1); and controlling each of a plurality of radio frequency coils (121a, 121b) that receive a nuclear magnetic resonance signal generated from a subject by an influence of a radio frequency pulse transmitted to the subject, the subject being placed in the static magnetic field having a magnetic field strength that changes spatially, thereby receiving the nuclear magnetic resonance signal at each of a plurality of frequencies tuned according to at least a distribution of the static magnetic field.

# FIG.1

EP 4 092 438 A1

# FIG.2

# FIG.3

# FIG.4

# FIG.5

120a     Ra     Rb

RF COIL

RF COIL

120b

# FIG.6

Ra  Rb

FREQUENCY
(MHz)

# FIG.7

EP 4 092 438 A1

# FIG.8A

# FIG.8B

EP 4 092 438 A1

# FIG.9

# FIG.10

EP 4 092 438 A1

# FIG.11

EP 4 092 438 A1

# FIG.12

EP 4 092 438 A1

FIG.13

PROC-ESSING CIRCUITRY 15

PULSE GENER-ATOR 161

ADC 473

DAC 162

DETEC-TOR 472

SYNTHE-SIZER 164

MODU-LATOR 465

CHANGE-OVER SWITCH 474

CHANGE-OVER SWITCH 467

171a

171b

166a

166b

RECEIVER COIL 122a

RECEIVER COIL 122b

TRANS-MITTER COIL 121a

TRANS-MITTER COIL 121b

RF PULSE OR NMR SIGNAL
CONTROL SIGNAL

# FIG.14

EP 4 092 438 A1

# FIG.15

EP 4 092 438 A1

# FIG.16

EP 4 092 438 A1

# FIG.17

RF PULSE OR NMR SIGNAL

CONTROL SIGNAL

15 — PROCESSING CIRCUITRY

161 — PULSE GENERATOR

162 — DAC

473 — ADC

472 — DETECTOR

164 — SYNTHESIZER

465 — MODULATOR

474 — CHANGEOVER SWITCH

467 — CHANGEOVER SWITCH

871a

871b

866a

866b

823 — TRANSMITTER/RECEIVER COIL

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/198396 A1 (PROMAXO INC [US]) 1 October 2020 (2020-10-01) <br> * paragraph [0108] - paragraph [0110] * <br> * paragraph [0115] - paragraph [0116] * <br> * paragraph [0156] - paragraph [0186] * <br> * figures 6A, 8, 10 * <br> ----- | 1-6,8-13 | INV. <br> G01R33/36 <br> G01R33/44 <br> G01R33/483 |
| X | US 2014/043029 A1 (BIBER STEPHAN [DE] ET AL) 13 February 2014 (2014-02-13) <br> * paragraph [0035] - paragraph [0040] * <br> * figures 1, 2 * <br> ----- | 1-6,8-13 | |
| X | US 2013/234706 A1 (MANDAL SOUMYAJIT [US] ET AL) 12 September 2013 (2013-09-12) <br> * paragraph [0054] - paragraph [0071] * <br> * paragraph [0137] * <br> * paragraph [0145] - paragraph [0167] * <br> * figures 1-3, 32A, 33 * <br> ----- | 1-6, 10-13 | |
| | | | TECHNICAL FIELDS SEARCHED (IPC) <br><br> G01R |

The present search report has been drawn up for all claims

| Place of search | Date of completion of the search | Examiner |
|---|---|---|
| Munich | 5 October 2022 | Streif, Jörg Ulrich |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 4023

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2022

| Patent document cited in search report | | Publication date | Patent family member(s) | | Publication date |
|---|---|---|---|---|---|
| WO 2020198396 | A1 | 01-10-2020 | AU 2020248421 | A1 | 30-09-2021 |
| | | | CA 3133107 | A1 | 01-10-2020 |
| | | | CN 113766875 | A | 07-12-2021 |
| | | | EP 3946042 | A1 | 09-02-2022 |
| | | | IL 286286 | A | 31-10-2021 |
| | | | JP 7133725 | B2 | 08-09-2022 |
| | | | JP 2022521810 | A | 12-04-2022 |
| | | | KR 20220002880 | A | 07-01-2022 |
| | | | KR 20220123319 | A | 06-09-2022 |
| | | | SG 11202109926U | A | 28-10-2021 |
| | | | US 2022155390 | A1 | 19-05-2022 |
| | | | WO 2020198396 | A1 | 01-10-2020 |
| US 2014043029 | A1 | 13-02-2014 | CN 103576108 | A | 12-02-2014 |
| | | | DE 102012213995 | B3 | 12-06-2014 |
| | | | KR 20140019757 | A | 17-02-2014 |
| | | | US 2014043029 | A1 | 13-02-2014 |
| US 2013234706 | A1 | 12-09-2013 | BR 102013005485 | A2 | 04-08-2015 |
| | | | US 2013234704 | A1 | 12-09-2013 |
| | | | US 2013234705 | A1 | 12-09-2013 |
| | | | US 2013234706 | A1 | 12-09-2013 |

EPO FORM P0459

For more details about this annex : see Official Journal of the European Patent Office, No. 12/82